(19) Europäisches Patentamt
European Patent Office
Office européen des brevets

(11) Publication number: **0 085 327**
**B1**

# (12) EUROPEAN PATENT SPECIFICATION

(45) Date of publication of patent specification: **30.04.86**

(51) Int. Cl.$^4$: **A 61 B 5/04**, A 61 N 1/04, H 01 B 1/12, C 09 J 3/14

(21) Application number: **83100323.1**

(22) Date of filing: **17.01.83**

## (54) Electrically conductive compositions and electrodes utilizing same.

(30) Priority: **18.01.82 US 340098**

(43) Date of publication of application:
**10.08.83 Bulletin 83/32**

(45) Publication of the grant of the patent:
**30.04.86 Bulletin 86/18**

(84) Designated Contracting States:
**DE FR GB NL SE**

(56) References cited:
**EP-A-0 012 402**
**WO-A-81/00785**
**WO-A-81/02097**
**US-A-4 066 078**
**US-A-4 136 078**
**US-A-4 273 135**

**MODERN PLASTICS INT., October 1981, vol. 11, nr. 10, Lausanne L.H. SPERLING "Interpenetrating polymer networks: now thermoplastic", pages 68-71**

(73) Proprietor: **MEDTRONIC, INC.**
**3055 Old Highway Eight**
**Minneapolis Minnesota 55440 (US)**

(72) Inventor: **Cahalan, Patrick T.**
**129 W. Eagle Lake Drive**
**Maple Grove Minnesota 55369 (US)**
Inventor: **Perrault, James J.**
**1707 69th Avenue North**
**Brocklyn Center Minnesota 55430 (US)**
Inventor: **Jevne, Allan H.**
**1314 153rd Lane N.E.**
**Anoka, Minnesota 55303 (US)**

(74) Representative: **Schwan, Gerhard, Dipl.-Ing.**
**Elfenstrasse 32**
**D-8000 München 83 (DE)**

**EP 0 085 327 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of prior art

This invention relates to electrically conductive compositions per se and electrodes utilizing the compositions as a conductive interface. More particularly, the invention relates to biomedical electrodes utilizing the conductive compositions described herein for establishing electrical contact with the skin of a subject. Consequently, in its preferred embodiments, this invention is directed to medical electrodes for application to the skin. Skin electrodes are of varying types and may be used either as transmission electrodes or as sensing or monitoring electrodes. A wide variety of design configurations have been provided in the past for these kinds of electrodes, all of which are applicable to this invention.

A variety of electrically conductive compositions for biomedical electrodes is known in the art. Generally, they comprise pastes, creams or gels which are relied upon to conduct electric current and improve or establish the electrical connection between the skin of a subject and the electrode per se. Hydrogels have been particularly popular for the conductive composition. Some hydrogels are even self-adhesive in that they are inherently tacky.

None of the known compositions is completely satisfactory from all standpoints i.e., as to electrical conductivity, adhesiveness, cohesiveness, flexibility, etc.

The electrically conductive compositions of this invention are more satisfactory overall than the prior art compositions. Additionally, they do not tend to dry out as readily. They are based on a unique polymer configuration known as an interpenetrating polymer network. Such networks have not been considered heretofore for use in forming conductive compositions, particularly in the preferred use herein i.e., biomedical electrodes.

For purposes herein, an interpenetrating polymer network is a combination of two polymers in network form, which occupy a specific volume and provide a single phase but are essentially not connected by primary chemical bonds. The networks referred to herein are prepared by synthesizing a crosslinked polymer from a water soluble monomer in the presence of an aqueous solution of a water soluble polymer, the first polymer being present in a predominant amount in the resultant interpenetrating polymer network relative to the amount of the second polymer.

Additional information concerning interpenetrating networks may be found in the article "Interpenetrating Polymer Networks: Now Thermoplastic" by L. H. Sperling, published in *Modern Plastics*, October, 1981, the content of which is hereby incorporated by reference.

The term "conformable" as used herein refers generally to the flexibility of the bulk conductive compositions or materials of the invention. The materials must be sufficiently flexible to conform to the surface of the skin or the like to which they or the electrode is attached and to provide a high surface area of contact to the skin or the like.

The term "cohesive" refers to the internal integrity of the conductive compositions or materials. Generally, the conductive material is self-supporting and must have more cohesive strength than adhesion to the skin or the like so that, when the electrode is removed from the skin or the like, the conductive material remains intact and does not leave an objectionable residue on the skin.

Summary of the invention

The conductive compositions of the invention essentially comprise an interpenetrating polymer network including a hydrophyllic, crosslinked polymer, preferably of an N-sulfohydrocarbon-substituted acrylamide, and an organic, hydrophilic, non-crosslinked polymer blended with a humectant and water. Preferably, the molecular weight of this polymer will be about at least 1800, about 5000 or 5100 being especially convenient, but molecular weight is not critical and may vary over a wide range.

Two of the essential components of the compositions of the invention form the required interpenetrating polymer network (IPN). These constituents are the aforementioned polymers.

The first polymer or polymeric constituent is at least one (usually only one) hydrophilic, crosslinked polymer, preferably of an N-sulfohydrocarbon-substituted acrylamide of the type described in U.S. Patent 4,136,078, which is incorporated herein by reference in respect of said acrylamide polymers. These polymers contain at least one polymer unit represented by the formula:

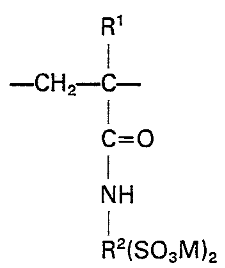

in which $R^1$ is hydrogen or a lower (as defined hereinafter) alkyl group and $R^2$ is a divalent or trivalent hydrocarbon group. As used herein, the term "hydrocarbon group" includes aliphatic, cycloaliphatic and

aromatic (including aliphatic and cycloaliphatic substituted aromatic and aromatic-substituted aliphatic and cycloaliphatic) groups. It also includes cyclic groups wherein the ring is completed through another portion of the molecule; that is, any two indicated substituents may together form a cyclic hydrocarbon group.

Substituted hydrocarbon, alkylaryl, alkylene, arylene, etc., groups are considered fully equivalent to the hydrocarbon, alkyl, aryl, alkylene, arylene, etc., groups and to be part of this invention. By "substituted" is meant groups containing substituents which do not alter significantly the character of reactivity of the group.

Preferably, the hydrocarbon or substituted hydrocarbon groups in the N-sulfohydrocarbon-substituted acrylamides are free from ethylenic and acetylenic unsaturation and have no more than about 30 carbon atoms, desirably no more than about 12 carbon atoms. Lower hydrocarbon groups are particularly preferred, the word "lower" denoting groups containing up to seven carbon atoms. Still more preferably, they are lower alkylene or arylene groups, most often alkylene.

In the formula, M is hydrogen or one equivalent of a cation and is usually hydrogen or an alkali metal. $R^1$ is hydrogen or lower alkyl as already defined, but is preferably hydrogen or methyl, usually hydrogen. $R^2$ may be any divalent or trivalent hydrocarbon group, preferably lower alkylene or arylene and usually lower alkylene. In a preferred embodiment of this invention, $R^2$ is

$$-\overset{R^3}{\underset{R^4}{\overset{|}{\underset{|}{C}}}}-CH_2-$$

wherein $R^3$ is hydrogen or a lower alkyl group, $R^4$ is a lower alkyl group and the sulfonic acid group is attached to the unsubstituted methylene carbon. These polymers may be obtained by the polymerization, either alone or in combination with other polymerizable vinyl monomers, of the corresponding monomeric N-sulfohydrocarbon-substituted acylamides of which the following acids, and their salts, are examples.

2-Acrylamidoethanesulfonic acid

$CH_2{=}CHCONHCH_2CH_2SO_3H$

2-Acrylamidopropanesulfonic acid

$CH_2{=}CHCONH\underset{\underset{CH_3}{|}}{C}HCH_2SO_3H$

2-Acrylamido-2-methylpropanesulfonic acid

$CH_2{=}CHCONH\overset{\overset{CH_3}{|}}{\underset{\underset{CH_2}{|}}{C}}{-}CH_2SO_3H$

3-Methacrylamidopropanesulfonic acid

$CH_2{=}\underset{\underset{CH_3}{|}}{C}{-}CONHCH_2CH_2CH_2SO_3H$

4-Methacrylamidocyclohexanesulfonic acid

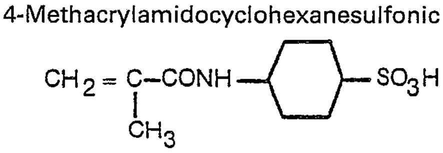

2-Acrylamido-2-phenylethanesulfonic acid

$CH_2{=}CHCONH\underset{\underset{C_6H_5}{|}}{C}HCH_2SO_3H$

2-Acrylamido-2-phenylpropanesulfonic acid

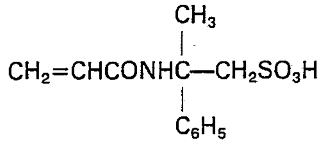

4-Acrylamidobenzenesulfonic acid

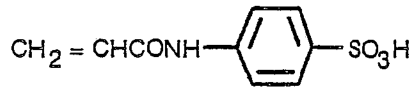

5-Acrylamidobenzene-1,3-disulfonic acid

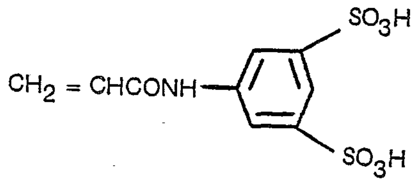

From the standpoint of economy, ease of preparation and polymerization, and effectiveness, the most desirable polymers are those of 2-acrylamido-2-methylpropanesulfonic acid or its salts, the use of which in medical electrodes is disclosed in EP—A—12402. The term "N-sulfohydrocarbon-substituted acrylamide" when used hereinafter will refer to this class of compounds generally, with the understanding that the above named acid and its salts, particularly the sodium and potassium salts are especially preferred.

The N-sulfohydrocarbon-substituted acrylamide polymers used in the compositions of this invention may be homopolymers or copolymers, the latter containing at least about 5% by weight, and preferably at least about 50%, of N-sulfohydrocarbon-substituted acrylamide units. The identity of the other monomer or monomers is not critical except that the polymer must be water-soluble or capable of forming a stable aqueous suspension. The most useful polymers are homopolymers (which are preferred) and copolymers with about 5—95%, preferably about 5—50% and most desirably about 5—30%, of an unsaturated acid (e.g., maleic acid) or a derivative thereof, especially an acrylic monomer such as acrylic or methacrylic acid or a salt or amide thereof, notably acrylamide, methacrylamide, N-methylacrylamide, diacetone acrylamide and the like.

The N-sulfohydrocarbon-substituted acrylamide polymer is prepared in aqueous solution. The polymerization may be promoted by typical initiators used in aqueous systems, especially peroxides, persulfates, persulfate-bisulfite and the like. The alkali metal salts of 2-acrylamido-2-methylpropanesulfonic acid may be polymerized in the absence of polymerization initiator.

It is sometimes advantageous to carry out the polymerization in the presence of a small amount of chain transfer agent, which tends to cause formation of a polymer with more uniformity in molecular weight than is otherwise produced. Suitable chain transfer agents are known to those skilled in the art.

Preferably, the first polymeric constituent will possess inherent tackiness, as do the N-sulfohydrocarbon-substituted acrylamides, but this is not necessary. Examples of other polymers which may be used as the first polymeric constituent are: hydroxymethylmethacrylate and potassium acrylate polymers.

The second polymeric constituent of the compositions and of the interpenetrating polymer network of this invention comprises an organic, non-crosslinked, hydrophilic polymer which possesses inherent tackiness. The most preferred non-crosslinked polymer of this type is polyacrylic acid, available commercially as an aqueous 50% solution Goodrite® K-732. Other examples of polymers useful as the second polymeric constituent are: polyvinylpyrollidone, and the various N-sulfohydrocarbon-substituted acrylamide polymers referred to above in non-crosslinked form.

The inclusion of the tacky non-crosslinked, hydrophilic second polymer constituent in the interpenetrating polymer network provides the tacky quality of the compositions of the invention along with any tackiness which may be provided by the first polymeric constituent. Preferably, however, the first polymeric constituent is also tacky as already pointed out.

As already indicated, the interpenetrating polymer network is formed by polymerizing and crosslinking the first polymeric constituent in the presence of the second polymeric constituent, as is described in detail hereinbelow.

In addition to the interpenetrating polymer network formed by the first and second polymeric constituents as described above, the compositions of the invention also essentially include a humectant. Applicable and preferred humectants include glycerol, propylene glycol, sorbitol, polyethylene glycol. Other non-volatile alcohols may be used as the humectant. The only requirement of the humectant is that it be compatible with the material and that it maintain a range of moisture content such that the stated properties of adhesiveness, conformability and conductivity be maintained.

The balance of the compositions of the invention essentially comprise water which need not be

distilled or deionized or the like although that is preferred. Any water is acceptable although it should at least be potable for medical uses. Ordinarily, the water content of a composition according to the invention will be on the order of about 12 to 45% by weight after the composition has been prepared and dried. During preparation in a preferred way, the water content is deliberately arranged to be higher for ease of preparation and is brought down to the desired range by drying, preferably oven drying. Also, when exposed to high humidity conditions, the compositions will absorb water and may increase the content thereof to as high as 50%. Consequently, when water content is referred to herein, unless otherwise specified, the water content being referred to is that of the composition as initially prepared and dried.

Generally, in preparing the compositions of the invention, the humectant and water may be included so as to be present during the formation of the interpenetrating polymer network.

The compositions of the invention will include the essential constituents in the following ranges (% by weight used throughout).

Interpenetrating polymer network (IPN) about 25—75%, about 55% preferred.

Humectant—about 5—50%, about 30% preferred.
Water—the balance.

As to the relative amounts of the two polymer constituents in the IPN, the first constituent is most preferably present from about 80% to about 90% and the second constituent is most preferably present from about 10% to about 20%. However, generally speaking, the first constituent must be present in an amount of at least about 35% in the IPN, amounts greater than about 50% being more preferred.

As to the relative amounts of the two polymeric constituents in terms of the overall composition, it is preferred that the first polymeric constituent be present within the range of about 24% to 74%, about 45% preferred, the second being present within the range of about 1% to 40%, about 10% preferred.

The compositions also include minor amounts of catalytic initiators which are insignificant as to the final properties of these compositions.

As is apparent to those skilled in the art, specific minimum amounts and specific relative amounts of the constituents of the compositions of the invention will vary considerably depending on the specific constituents combined and the purpose for which the composition is to be used. In general, it is necessary to select those amounts which will provide a cohesive, flexible, conductive, self-supporting, conformable body of material suitable to a particular intended purpose.

In addition to the interpenetrating polymer network, humectant, water i.e., the essential constituents, and the various initiators, the compositions of this invention may contain other compatible ingredients which modify the characteristics thereof, e.g., by improving their remoistening ability, modifying their setting speed, and increasing their thermal stability. These additives may include fillers such as clay, chalk, fiberglass and carbon and antioxidants such as butylated hydroxyanisole, butylated hydroxytoluene and sodium benzoate, as well as others.

As already stated, for interface use with biomedical electrodes, the most preferred embodiment of the invention uses, as the crosslinked polymer of the interpenetrating polymer network, a salt of polymerised 2-acrylamido-2-methylpropanesulfonic acid, sodium, potassium and lithium salts being typical, the sodium salt being most readily available commercially at the present time. The preferred organic, non-crosslinked hydrophilic polymer is polyacrylic acid. The preferred humectant is glycerol.

Compositions including these constituents within the following ranges are preferred for biomedical electrode use (% expressed in terms of overall composition):

| | |
|---|---|
| polymerized 2-acrylamido-2-methylpropanesulfonic acid or one of its salts, sodium salt most preferred about 24—74%, about 45% preferred<br><br>polyacrylic acid about 1—40%, about 10% preferred | } IPN% overall about 25%—75% |

glycerol about 5—50%, about 30% preferred

methylene bis-acrylamide cross-linking agent and initiators . . . up to about 5%, about 0.1% preferred

water . . . the balance

Brief description of the drawings

Fig. 1 is a schematic perspective view of an electrode according to the invention.
Fig. 2 is a plan view of the electrode of Fig. 1.
Fig. 3 is a cross-sectional view taken along line 3—3 of Fig. 2.
Fig. 4 is an elevational view of a preferred electrode.
Fig. 5 is a plan view of the electrode shown in Fig. 4.

Description of specific preferred embodiments

The preferred usage of the compositions of the invention lies in their use as the interfacing material for biomedical electrodes. In this usage the compositions provide efficient and effective signal transmission media between a subject's skin and various kinds of electro-medical apparatus.

The compositions of the invention and electrodes utilizing same are intended to possess inherent adhesive properties for maintaining contact with the skin as well as possessing a certain amount of flexibility and conformability for movement with the skin in addition to a uniform configuration and homogeneity of composition for contact with the skin and the passage of uniform current densities to or from the skin. The electrodes utilizing the compositions of the invention are easily handled, have an effective operating life without substantial drying out and are non-irritating to the subject.

As previously pointed out, this invention particularly lends itself to medical electrodes of varying types, shapes and configurations. For exemplary purposes herein, a skin electrode 10 is shown in one of the common rectangular configurations.

Electrode 10 includes flexible, adhesive and conductive member 12 for contacting the skin. The composition of member 12 is in accordance with this invention. An electrical lead means 14, including a conductive member 14a and an insulating sheath or covering 14b, electrically contacts member 12.

The embodiment of the electrode as shown in the Figures 1—3 also includes a support or backing 18, the chief purpose of which is to provide a protective and supportive member for the substrate. A preferred backing material is polyethylene foam. One such material is commercially available from Fasson, Inc., a division of Avery International of Paynesville, Ohio, under the trade designation MED 416. The material is a four pound density cross-linked polyethylene foam coated with a tacky adhesive material of an acrylic type. The foam is 1/16th of an inch thick. However, various thicknesses may be used. The foam need not be coated with the adhesive since it will in most instances, readily adhere to substrate 12 which, as previously pointed out, is inherently adhesive.

The preferred embodiment makes used of the adhesive coated foam as backing 18, the stainless steel foil current distribution member 16 and substrate member 12. The substrate member may be of various thicknesses, about .05 to .250 inches being preferred. Greater thicknesses may be used as dimension is not critical so long as the electrical resistance is not excessive for the particular use involved.

In operation and use, electrode 10 is applied with conductive substrate 12 in direct contact with the skin. The adhesive properties of substrate 12 eliminate the need for separate adhesive tape or any other separate securing measures to hold electrode 10 in continuous contact with the skin. Upon prolonged exposure or use, the substrate may be wiped with water or alcohol to increase its adhesiveness. Electrical signals either to or from the skin, depending on the type of electrode application desired, are conducted through substrate member 12, the current distribution member 16 and electrical lead means 14 including wire 14a. Preferably, wire 14a contacts distribution member 16 by being held between it and backing 18.

In a transmission type of arrangement, lead 14 receives electrical signals from an external apparatus (not shown). This signals are conducted into the current distribution member 16 which in turn conducts them into the conductive substrate 12. In this manner current densities are uniformly distributed over the area of substrate 12 and in turn uniformly transmitted to the skin surface in contact with substrate 12. In a sensing or monitoring arrangement, the flow of electricity is reversed in direction, originating at the skin and being conducted through the substrate 12, distribution member 16, lead wire 14a and to a suitable electro-medical monitoring apparatus (not shown).

Referring now to Figures 4 and 5, a preferred electrode configuration is shown. The electrode includes as the backing a conductive, carbon-filled silicone rubber support body 18 including a raised rib 18a contacted by an electrical lead 14. Attached to the bottom of body 18 is substrate 12 which comprises a composition of the invention. The composition is constituted so as to be adhesive, conductive, pliant, conformable and cohesive. Attachment is by the inherent adhesive quality of the composition.

Typically, about 50% carbon mixed with the silicone rubber will provide a suitable support body although this amount is not critical. Any amount providing a conductive body is generally satisfactory.

Other conductive fillers such as silver and the like may be used. Also, materials other than silicone rubber may be used such as any elastomer material which is either conductive inherently or which may be rendered conductive by filling.

Also, the support member 18 may be a thin flexible conductive material such as an aluminum or stainless steel foil or the like to which substrate 12 is directly attached, lead 14 then being attached to the foil member.

The composition of conductive substrate member 12 is unique to the invention, as already indicated. As previously stated, it includes an interpenetrating polymer network, preferably of polymerized 2-acrylamido-2-methylpropanesulfonic (AMPS) acid or one of its salts and polyacrylic acid. In addition to the interpenetrating polymer network, the composition includes a humectant, preferably glycerol, and water so as to provide in preferred form a flexible sheet-like body. Crosslinked copolymers may also be used as the first polymeric constituent to form the interpenetrating network. Copolymers may also be used as the second polymeric constituent. Additional polymeric thickeners and the like may be blended into the composition, although they are not ordinarily needed. The various constituents are provided in such relative amounts as to form a flexible, self-supporting material with substantial shape retention which has adhesive properties and which is electrically conductive.

Preferably, the interpenetrating polymer network is formed by polymerizing and crosslinking 2-acrylamido-2-methylpropanesulfonic acid or one of its salts, most preferably the sodium salt form, in the presence of non-cross-linked polyacrylic acid. The polymerization is carried out in aqueous solution in the presence of the humectant.

In the principle preferred embodiments, substrate member 12 comprises a sheet-like body of interpenetrating polymer network material formed from the sodium salt of 2-acrylamido-2-methylpropanesulfonic acid monomer purchased from The Lubrizol Corporation. The monomer upon being dissolved in water is readily polymerized. The preferred non-crosslinked polymer is polyacrylic acid dissolved in water. The preferred humectant is glycerol mixed in the aqueous solution in which polymerization and crosslinking of the first polymeric constituent is carried out in the presence of the second polymeric constituent.

The following Examples are provided for illustration of the invention.

Example I

140 mls of sodium 2-acrylamido-2-methylpropanesulfonate (NaAMPS) solution and 18 mls of Goodrite® K-732 solution are mixed together. The Na AMPS solution comprises 35.70% Na AMPS, 21% glycerol and 0.76% methylene bis-acrylamide (MBA) (crosslinking agent), balance water. The Goodrite® K-732 solution comprises 50% by weight polyacrylic acid in water. The molecular weight of the polyacrylic acid in Goodrite® K-732 is 5100. The mixture is purged with nitrogen for about ten minutes following which catalytic initiators are added. Preferred catalytic initiators comprise potassium metabisulfite solution (99.62% deionized water, 0.38% potassium meta-bisulfite) potassium persulfate solution (99.62% deionized water, 0.38% potassium persulfate) and ferrous sulfate solution (99.76% deionized water, 0.24% ferrous sulfate). The catalytic initiators are simultaneously injected, via individual 3 cc syringes into the nitrogen purged agitated monomer solution. Upon addition of the initiators, the mixture is allowed to agitate for a short time, approximately ten seconds is satisfactory, then it is quickly poured into a tray or other suitable mold in an enclosed nitrogen atmosphere. The solution polymerizes to a solid, tacky, clear gel like material in about 5 minutes following exotherm. The exotherm time is about 90±15 seconds.

Preferably, the tray or mold (7-3/4×12-½ inches used in this instance) into which the solution is poured will have the bottom thereof covered with a Mylar polyester sheet. Following polymerization the polymer body may then be readily removed from the mold along with the Mylar® polyester sheet as a backing thereof.

The body is then dried with the Mylar in place in a forced air oven. For this particular example, drying at about 3 hours at 70°C is adequate.

The preparation described provides a sheet-like body 7-3/8×12 inches and about .060 inches thick having a composition after drying as follows (% in terms of overall composition):

| | |
|---|---|
| Na AMPS | 49.2% |
| Polyacrylic acid | 8.9% |
| Glycerol | 28.9% |
| MBA (including initiators) | 0.1% |
| Water | 12.9% |

The volume resistivity of this material and all other materials reported herein can be expected to fall below about 30,000 ohm-cm.

Example I represents the most preferred composition and the most preferred mode of preparation, utilizing prepared solutions which are mixed together. This technique does not utilize heat activated catalysts, which may be used if desired. Non-heat activated catalysts are preferred from a production standpoint.

Other modes of preparation are illustrated in some of the following Examples which do use heat activated catalysts.

Example II

The following constituents were mixed together under ambient conditions. The mixture was poured into a mold. The mold was placed in a vacuum oven, deaerated and heated at a temperature of 65°C for about 4 hours. It was then placed in a forced air oven and dried at about 60°C to a weight of about 60 gms.

| Mixture | |
|---|---|
| Hydroxyethylmethacrylate* (HEMA) | 15 gms |
| Polyvinylpyrrolidone (PVP) | 24 gms |
| Glycerol | 10 gms |
| Water | 50 gms |

2,2'-Azobis[2-methylpropionitrile] (AIBN) 0.02 gms

* sold with 0.5—1.5% impurity ethyleneglycoldimethacrylate, a crosslinker.

The composition of the resultant product is:

| Final composition | |
|---|---|
| HEMA | 25% |
| PVP | 40% |
| Glycerol | 16.7% |
| AIBN | 0.03% |
| Water | 18.27% |

Example III

Same as Example II with differences noted.

| Mixture | |
|---|---|
| Potassium acrylate | 10 gms (57% solution in water) |
| Goodrite® K-732 | 0.5 gms |
| Glycerol | 10 gms |
| MBA | 10 gms (1% solution in water) |
| Ammonium persulfate (AP) | 0.5 gms (2% solution in water) |

In preparation, this material was oven dried to a weight of 20 gms.

| Final composition | |
|---|---|
| Potassium acrylate | 28.5% |
| Goodrite® K-732 | 1.2% |
| Glycerol | 50.5% |
| MBA | 0.5% |
| AP | 0.05% |
| Water | 19.75% |

Example IV

Same as Example III with differences noted.

Mixture

| | |
|---|---|
| Na AMPS | 150 gms (50% solution in water) |
| Goodrite® K-732 (MW 1800) PAA) | 9 gms (50% solution in water) |
| Glycerol | 5 gms |
| MBA | 50 gms (1% solution in water) |
| AP | 4 gms (2% solution in water) |

Dried to 100 gms.

Final composition

| | |
|---|---|
| Na AMPS | 75% |
| Goodrite® K-732 (PAA) | 4.5% |
| Glycerol | 5% |
| MBA | 0.5% |
| AP | 0.08% |
| Water | 14.92% |

Example V

Same as Example III with differences noted.

Mixture

| | |
|---|---|
| Na AMPS solution as in Example I | 50 mls |
| Carbopol 940 (tradename of B. F. Goodrich Co.) (PAA MW 4 million) | 1.5 gms |
| AP | 0.5 gms |

Dried to 33 gms.

Final composition

| | |
|---|---|
| Na AMPS | 51.9% |
| PAA | 4.5% |
| Glycerol | 30.5% |
| MBA | 1.0% |
| AP | 0.03% |
| Water | 12.07% |

Example VI

Same as Example III with differences noted.

| Mixture | |
|---|---|
| Na AMPS | 84 gms (50% solution in water) |
| Acrylamide (copolymer with Na AMPS) | 40 gms |
| PVP | 6 gms |
| Glycerol | 40 gms |
| MBA | 6 gms (1% solution in water) |
| AP | 2 gms |

No drying—as made.

| Final composition | |
|---|---|
| Na AMPS | 30.3% |
| Acrylamide | 1.9% |
| PVP | 4.3% |
| Glycerol | 28.8% |
| MBA | 0.04% |
| AP | 0.03% |
| Water | 34.63% |

Example VII

Same as Example III with differences noted.

| Mixture | |
|---|---|
| Na AMPS solution as in Example I | 10 gms |
| Goodrite® K-732 (PAA) | 2 gms |
| MBA | 40 gms (1% solution in water) |
| AP | 0.5 gms |

Dried to 9.5 gms.

| Final composition | |
|---|---|
| Na AMPS | 37.6% |
| PAA | 10.5% |
| Glycerol | 22.1% |
| MBA | 5.0% |
| AP | 0.1% |
| Water | 24.7% |

Example VIII

Same as Example III with differences noted.

| | Mixture |
|---|---|
| Na AMPS | 80 gms (50% solution in water) |
| Potassium acrylate (copolymer with Na AMPS) | 20 gms (57% solution in water) |
| PVP | 6 gms |
| Glycerol | 40 gms |
| MBA | 6 gms |
| AIBN | 0.02 gms |

Not dried—as made.

| | Final composition |
|---|---|
| Na AMPS | 26% |
| Potassium acrylate | 7.5% |
| PVP | 3.9% |
| Glycerol | 26.3% |
| MBA | 0.04% |
| AIBN | 0.01% |
| Water | 36.25% |

Example IX

Same as Example III with differences noted.

| | Mixture |
|---|---|
| 2-Acrylamido-2-methylpropane-sulfonic acid (AMPS) | 80 gms (50% solution in water) |
| PVP | 6 gms |
| Glycerol | 40 gms |
| MBA | 6 gms (1% solution in water) |
| AP | 0.4 gms (1% solution in water) |

Not dried—as made.

| | Final composition |
|---|---|
| AMPS | 30.2% |
| PVP | 4.5% |
| Glycerol | 30.2% |
| MBA | 0.045% |
| AP | 0.003% |
| Water | 35.052% |

Example X

Same as Example I with differences noted.

Mixture

| | |
|---|---|
| Na AMPS | 85 gms (50% solution in water) |
| Goodrite® K-732 | 15 gms (50% solution in water) |
| Glycerol | 25 gms |
| MBA | 9 gms (1% solution in water) |
| Each catalyst in Example I | 2 gms each |
| NaCl | 0.75 gms |

Dried to 138 gms.

Final composition

| | |
|---|---|
| Na AMPS | 30.8% |
| PAA | 5.4% |
| Glycerol | 18.1% |
| MBA | 0.07% |
| Catalysts | 0.02% |
| NaCl | 0.54% |
| Water | 45.08% |

Example XI

Same as Example I with differences noted.

Mixture

| | |
|---|---|
| Na AMPS | 85 gms (50% solution in water) |
| Goodrite® K-732 (PAA) | 20 gms (50% solution in water) |
| Propylene glycol | 15 gms |
| MBA | 2 gms |
| Catalysts as in Example I | 6 gms total |

Not dried—as made.

Final composition

| | |
|---|---|
| Na AMPS | 29.7% |
| PAA | 7.0% |
| Propylene glycol | 10.5% |
| Glycerol | 10.5% |
| MBA | 0.014% |
| Catalysts | 0.014% |
| Water | 42.272% |

As already pointed out, various additives may be included in the compositions of the invention, it only

being necessary that the compositions include, in varying amounts, the essential interpenetrating polymer network with a sufficient amount of water and humectant to provide the requisite electrically conductive, conformable and flexible, cohesive, adhesive body. Various body thicknesses of the compositions may be used as desired and any electrode configuration with or without backing support and current distribution member may be used. Release paper of the waxed, silicone-treated or plastic coated variety or Mylar polyester sheet may be included to protect the substrate material prior to use.

Electrodes utilizing these compositions or any other devices utilizing same or the compositions per se should be stored in a sealed container or under controlled conditions or humidity to prevent drying out.

**Claims**

1. An electrically conductive, adhesive, cohesive composition characterized by:
an interpenetrating polymer network essentially including two components: a first polymeric constituent which is crosslinked and hydrophilic and a second polymeric constituent which is hydrophilic and not crosslinked, the network being comprised of at least about 35% of the first constituent;
humectant; and
water.

2. The composition of claim 1 wherein the interpenetrating network comprises an amount of the first polymeric constituent in excess of about 50% and preferably between about 80% and 90%.

3. The composition of claim 1 or 2 wherein the interpenetrating polymer network comprises about 25% to 70% of the overall composition.

4. The composition of any one of the preceding claims wherein the amount of the humectant is about 5% to 50%.

5. The composition of any one of the preceding claims wherein the first polymeric constituent is a hydroxymethylmethacrylate polymer.

6. The composition of any one of claims 1 to 4 wherein the first polymeric constituent is a potassium acrylate polymer.

7. The composition of any one of claims 1 to 4 wherein the first polymeric constituent is an N-sulfohydrocarbon substituted acrylamide polymer.

8. The composition of any one of claims 1 to 4 wherein the second polymeric constituent is polyacrylic acid.

9. An adhesive, electrically conductive composition, characterized by essentially including between about 25% and about 75% of an interpenetrating polymer network formed as the result of the polymerization and cross-linking of 2-acrylamido-2-methylpropanesulfonic acid or one of its salts in the presence of a hydrophilic polymer which is not cross-linked, and including between about 5% to about 50% of a humectant, the balance being substantially water.

10. The composition of claim 9 wherein a salt form of the 2-acrylamido-2-methylpropanesulfonic acid is polymerized.

11. The composition of claim 10 wherein the salt is a sodium salt.

12. The composition of any one of claims 9 to 11 wherein the amount of the polymer network is about 50%.

13. The composition of any one of claims 9 to 12 wherein the hydrophilic polymer is polyacrylic acid.

14. The composition of claim 13 wherein the molecular weight of the polyacrylic acid is about 5,000.

15. The composition of any one of the preceding claims wherein the humectant is glycerol.

16. The composition of any one of claims 1 to 14 wherein the humectant is propylene glycol, polyethylene glycol or sorbitol.

17. The composition of any one of the preceding claims wherein the amount of the humectant is about 30%.

18. An electrically conductive, adhesive, conformable and flexible, cohesive body, characterized by:
an interpenetrating polymer network essentially including two components: polymerized and crosslinked 2-acrylamido-2-methylpropanesulfonic acid, its salts or copolymers of the acid or its salts as the first component and, as the second component, an organic, hydrophilic polymer which is not cross-linked, the amounts of the first component and the second component in terms of overall composition ranging from about 24% to 74% and 1% to 40%, respectively;
a humectant ranging in amount from about 5% to 50%, and
water, substantially the balance.

19. The body of claim 18 wherein the amounts of the two polymer network components are about 45% and 10%, respectively.

20. The body of claim 18 or 19 wherein the amount of the humectant is about 30%.

21. The body of any one of claims 18 to 20 wherein the amount of the interpenetrating polymer network is about 25% to 75% and preferably about 50%.

22. The body of any one of claims 18 to 21 wherein the first component is a polymerized salt of 2-acrylamido-2-methylpropanesulfonic acid.

23. The body of claim 22 wherein the salt is a sodium salt.

24. The body of any one of claims 18 to 23 wherein the second component is polyacrylic acid.

25. The body of claim 24 wherein the molecular weight of the polyacrylic acid is about 5,000.

26. An electrically conductive, adhesive, cohesive body, characterized in that the body comprises predominately the composition according to claim 1.

27. The body of claim 26 wherein the interpenetrating polymer network comprises at least about 50% of the crosslinked polymer.

28. The body of claim 27 wherein the amount is about 80% to 90% and the second component comprises substantially the balance of the network.

29. The body of claim 14 wherein the humectant is glycerol.

30. The body of claim 14 wherein the humectant is propylene glycol, sorbitol or polyethylene glycol.

31. An adhesive electrode for establishing electrical contact with a surface, comprising:

support means and terminal means for connection to external electrical circuit means, and

an adhesive, conductive body as defined in claim 26 for electrically interfacing with the surface to be contacted, the body being contacted by the support means and being in electrical communication with the terminal means.

32. The electrode of claim 31 wherein the composition includes as the two components: polymerized crosslinked 2-acrylamido-2-methylpropanesulfonic acid, its salts or copolymers of the acid or its salts as the first component and an organic, non-crosslinked hydrophilic polymer, as the second polymeric component, the overall amounts of the first and second components ranging from about 24% to 74% and 1% to 40%, respectively.

33. The electrode of claim 32 wherein the first component is a polymerized salt form of the 2-acrylamido-2-methylpropanesulfonic acid.

34. The electrode of claim 33 wherein the salt is a sodium salt.

35. The electrode of any one of claims 32 to 34 wherein the hydrophilic polymer is polyacrylic acid.

36. The electrode of claim 35 wherein the molecular weight of the polyacrylic acid is about 5,000.

37. The electrode of any one of claims 31 to 36 wherein the humectant is glycerol.

38. The electrode of any one of claims 31 to 36 wherein the humectant is propylene glycol, sorbitol or polyethylene glycol.

39. The electrode of any one of claims 31 to 38 wherein the amount of the humectant is about 30%.

**Patentansprüche**

1. Elektrisch leitfähige, klebende, kohäsive Zusammensetzung, gekennzeichnet durch:

ein ineinandergeschachteltes Polymernetzwerk, das im wesentlichen zwei Komponenten aufweist, und zwar einen ersten Polymerbestandteil, der vernetzt und hydrophil ist, und einen zweiten Polymerbestandteil, der hydrophil und nicht vernetzt ist, wobei das Netzwerk mindestens etwa 35% des erste Bestandteils;

Feuchthaltemittel; und

Wasser enthält.

2. Zusammensetzung nach Anspruch 1, wobei das ineinandergeschachtelte Netzwerk eine Menge von über etwa 50% und vorzugsweise zwischen etwa 80% und 90% des ersten Polymerbestandteils enthält.

3. Zusammensetzung nach Anspruch 1 oder 2, wobei das ineinandergeschachtelte Polymernetzwerk etwa 25% bis 70% der gesamten Zusammensetzung ausmacht.

4. Zusammensetzung nach einem der vorhergehenden Ansprüche, wobei die Menge des Feuchthaltemittels etwa 5% bis 50% beträgt.

5. Zusammensetzung nach einem der vorhergehenden Ansprüche, wobei der erste Polymerbestandteil ein Hydroxymethylmethacrylatpolymer ist.

6. Zusammensetzung nach einem der Ansprüche 1 bis 4, wobei der erste Polymerbestandteil ein Kaliumacrylatpolymer ist.

7. Zusammensetzung nach einem der Ansprüche 1 bis 4, wobei der erste Polymerbestandteil ein N-schwefelkohlenwasserstoffsubstituiertes Acrylamidpolymer ist.

8. Zusammensetzung nach einem der Ansprüche 1 bis 4, wobei der zweite Polymerbestandteil Polyacrylsäure ist.

9. Klebende, elektrisch leitfähige Zusammensetzung, dadurch gekennzeichnet, daß sie im wesentlichen zwischen etwa 25% und etwa 75% eines ineinandergeschachtelten Polymernetzwerks, gebildet als Ergebnis der Polymerisation und Vernetzung von 2-Acrylamid-2-Methylpropansulfonsäure oder einer ihrer Salze in Gegenwart eines nicht vernetzten hydrophilen Polymers, und zwischen etwa 5% und etwa 50% eines Feuchthaltemittels aufweist, wobei der Rest im wesentlichen Wasser ist.

10. Zusammensetzung nach Anspruch 9, wobei ein Salz der 2-Acrylamid-2-Methylpropansulfonsäure polymerisiert ist.

11. Zusammensetzung nach Anspruch 10, wobei das Salz ein Natriumsalz ist.

12. Zusammensetzung nach einem der Ansprüche 9 bis 11, wobei dei Menge des Polymernetzwerks etwa 50% beträgt.

13. Zusammensetzung nach einem der Ansprüche 9 bis 12, wobei das hydrophile Polymer Polyacrylsäure ist.

14. Zusammensetzung nach Anspruch 13, wobei das Molekulargewicht der Polyacrylsäure etwa 5000 beträgt.

15. Zusammensetzung nach einem der vorhergehenden Ansprüche, wobei das Feuchthaltemittel Glyzerin ist.

16. Zusammensetzung nach einem der Ansprüche 1 bis 14, wobei das Feuchthaltemittel Propylenglykol, Polyethylenglykol oder Sorbit ist.

17. Zusammensetzung nach einem der vorhergehenden Ansprüche, wobei die Menge des Feuchthaltemittels etwa 30% beträgt.

18. Elektrisch, leitfähiger, klebender, anpassungsfähiger und flexibler, kohäsiver Körper, gekennzeichnet durch:

ein ineinandergeschachteltes Polymernetzwerk, das im wesentlichen zwei Komponenten aufweist, und zwar polymerisierte und vernetzte 2-Acrylamid-2-Methylpropansulfonsäure, deren Salze oder Copolymere der Säure oder ihrer Salze als die erste Komponente sowie ein organisches, hydrophiles, nicht vernetztes Polymer als die zweite Komponente, wobei die Anteile der ersten Komponente und der zweiten Komponente bezogen auf die Gesamtzusammensetzung zwischen etwa 24% und 74% bzw. 1% und 40% liegen;

ein Feuchthaltemittel in einer Menge von etwa 5% bis 50% und

im wesentlichen Wasser als Rest.

19. Körper nach Anspruch 18, wobei die Mengen der beiden Komponenten des Polymernetzwerks etwa 45% bzw. 10% betragen.

20. Körper nach Anspruch 18 oder 19, wobei die Menge des Feuchthaltemittels etwa 30% beträgt.

21. Körper nach einem der Ansprüche 18 bis 20, wobei die Menge des ineinandergeschachtelten Polymernetzwerks etwa 25% bis 75% und vorzugsweise etwa 50% beträgt.

22. Körper nach einem der Ansprüche 18 bis 21, wobei die erste Komponente ein polymerisiertes Salz der 2-Acrylamid-2-Methylpropansulfonsäure ist.

23. Körper nach Anspruch 22, wobei das Salz ein Natriumsalz ist.

24. Körper nach einem der Ansprüche 18 bis 23, wobei die zweite Komponente Polyacrylsäure ist.

25. Körper nach Anspruch 24, wobei das Molekulargewicht der Polyacrylsäure etwa 5000 beträgt.

26. Elektrisch leitfähiger, klebender, kohäsiver Körper, dadurch gekennzeichnet, daß der Körper überwiegend aus der Zusammensetzung nach Anspruch 1 besteht.

27. Körper nach Anspruch 26, wobei das ineinandergeschachtelte Polymernetzwerk mindestens etwa 50% des vernetzten Polymers enthält.

28. Körper nach Anspruch 27, wobei die Menge etwa 80% bis 90% beträgt und die zweite Komponente im wesentlichen den Rest des Netzwerks ausmacht.

29. Körper nach Anspruch 14, wobei das Feuchthaltemittel Glyzerol ist.

30. Körper nach Anspruch 14, wobei das Feuchthaltemittel Propylenglykol, Sorbit oder Polyethylenglykol ist.

31. Klebende Elektrode zur Herstellung eines elektrischen Kontakts mit einer Oberfläche, die aufweist:

eine Trägeranordnung und Anschlußmittel zur Verbindung mit einer externen elektrischen Schaltungsanordnung, sowie einen klebenden, leitfähigen Körper gemäß Anspruch 26, zur Ankopplung an die zu kontaktierende Oberfläche, wobei der Körper mit der Trägeranordnung in Kontakt sowie mit den Anschlußmitteln in elektrischer Verbindung steht.

32. Elektrode nach Anspruch 31, wobei die Zusammensetzung als die beiden Komponenten aufweist: polymerisierte vernetzte 2-Acrylamid-2-Methylpropansulfonsäure, deren Salze oder Copolymere der Säure oder deren Salze als die erste Komponente sowie ein organisches, nicht vernetztes hydrophiles Polymer als die zweite Polymerkomponente, wobei die Gesamtmengen der ersten und der zweiten Komponente zwischen etwa 24% und 74% bzw. 1% und 40% liegen.

33. Elektrode nach Anspruch 32, wobei die erste Komponente ein polymerisiertes Salz der 2-Acrylamid-2-Methylpropansulfonsäure ist.

34. Elektrode nach Anspruch 33, wobei das Salz ein Natriumsalz ist.

35. Elektrode nach einem der Ansprüche 32 bis 34, wobei das hydrophile Polymer Polyacrylsäure ist.

36. Elektrode nach Anspruch 35, wobei das Molekulargewicht der Polyacrylsäure etwa 5000 beträgt.

37. Elektrode nach einem der Ansprüche 31 bis 36, wobei das Feuchthaltemittel Glyzerol ist.

38. Elektrode nach einem der Ansprüche 31 bis 36, wobei das Feuchthaltemittel Propylenglykol, Sorbit oder Polyethylenglykol ist.

39. Elektrode nach einem der Ansprüche 31 bis 38, wobei die Menge des Feuchthaltemittels etwa 30% beträgt.

**Revendications**

1. Composition adhésive, cohérente, conductrice de l'électricité, caractérisée par:

un réseau de polymères en interpénétration comprenant essentiellement deux composants: un premier constituant polymère qui est réticulé et hydrophile, et un second constituant polymère qui est hydrophile et n'est pas réticulé, le réseau étant constitué d'au moins environ 35% du premier constituant:

un humectant; et

de l'eau.

2. Composition selon la revendication 1, dans laquelle le réseau de polymères en interpénétration comprend une proportion du premier constituant polymère supérieure à environ 50% et de préférence comprise entre environ 80% et 90%.

3. Composition selon la revendication 1 ou 2, dans laquelle le réseau de polymères en interpénétration constitue environ 25% à 70% de la composition totale.

4. Composition selon l'une quelconque des revendications précédentes, dans laquelle la proportion de l'humectant est d'environ 5% à 50%.

5. Composition selon l'une quelconque des revendications précédentes, dans laquelle le premier constituant polymère est un polymère de méthacrylate d'hydroxyméthyle.

6. Composition selon l'une quelconque des revendications 1 à 4, dans laquelle le premier constituant polymère est un polymère d'acrylate de potassium.

7. Composition selon l'une quelconque des revendications 1 à 4 dans laquelle le premier constituant polymère est un polymère d'acrylamide substitué sur N par un groupe sulfohydrocarboné.

8. Composition selon l'une quelconque des revendications 1 à 4, dans laquelle le second constituant polymère est un acide polyacrylique.

9. Composition adhésive conductrice de l'électricité, caractérisée en ce qu'elle comprend essentiellement environ 25% à environ 75% d'un réseau de polymères en interpénétration formé par suite de la polymérisation et de la réticulation d'acide 2-acrylamido-2-méthylpropane-sulfonique ou de l'un de ses sels en présence d'un polymère hydrophile qui n'est pas réticulé et en ce qu'elle comprend environ 5% à environ 50% d'un humectant, le reste consistant sensiblement en eau.

10. Composition selon la revendication 9, dans laquelle une forme sel de l'acide 2-acrylamido-2-méthylpropane-sulfonique est polymérisée.

11. Composition selon la revendication 10, dans laquelle le sel est un sel de sodium.

12. Composition selon l'une quelconque des revendications 9 à 11 dans laquelle la proportion du réseau de polymères est d'environ 50%.

13. Composition selon l'une quelconque des revendications 9 à 12 dans laquelle le polymère hydrophile est un acide polyacrylique.

14. Composition selon la revendication 13 dans laquelle le poids moléculaire de l'acide polyacrylique est d'environ 5000.

15. Composition selon l'une quelconque des revendications précédentes dans laquelle l'humectant est le glycérol.

16. Composition selon l'une quelconque des revendications 1 à 14 dans laquelle l'humectant est le propylène-glycol, le polyéthylène-glycol ou le sorbitol.

17. Composition selon l'une quelconque des revendications précédentes, dans laquelle la proportion de l'humectant est d'environ 30%.

18. Corps cohérent, adhésif, conformable et souple, conducteur de l'électricité, caractérisé par:

un réseau de polymères en interpénétration comprenant essentiellement deux composants: de l'acide 2-acrylamido-2-méthylpropane-sulfonique polymérisé et réticulé, ses sels ou des copolymères de l'acide ou de ses sels comme premier composant et, comme second composant, un polymère organique hydrophile qui n'est pas réticulé, les proportions du premier composant et du second composant, par rapport à la composition totale, étant d'environ 24% à 74% et de 1% à 40%, respectivement;

un humectant en proportion d'environ 5% à 50%, et

de l'eau constituant sensiblement le reste.

19. Corps selon la revendication 18, dans lequel les proportions des deux composants du réseau de polymères sont d'environ 45% et 10%, respectivement.

20. Corps selon la revendication 18 ou 19 dans lequel la proportion de l'humectant est d'environ 30%.

21. Corps selon l'une quelconque des revendications 18 à 20 dans lequel la proportion du réseau de polymères en interpénétration es d'environ 25% à 75%, et de préférence d'environ 50%.

22. Corps selon l'une quelconque des revendications 18 à 21, dans lequel le premier composant est un sel polymérisé de l'acide 2-acrylamido-2-méthylpropane-sulfonique.

23. Corps selon la revendication 22 dans lequel le sel est un sel de sodium.

24. Corps selon l'une quelconque des revendications 18 à 23 dans lequel le second composant est un acide polyacrylique.

25. Corps selon la revendication 24 dans lequel le poids moléculaire de l'acide polyacrylique est d'environ 5000.

26. Corps cohérent, adhésif, conducteur de l'électricité, caractérisé en ce que le corps comprend de façon prédominante la composition selon la revendication 1.

27. Corps selon la revendication 26 dans lequel le réseau de polymères en interpénétration comprend au moins environ 50% du polymère réticulé.

28. Corps selon la revendication 27 dans lequel la proportion est d'environ 80% à 90% et le second composant constitue sensiblement le reste du réseau.

29. Corps selon la revendication 14 dans lequel l'humectant est le glycérol.

30. Corps selon la revendication 14 dans lequel l'humectant est le propylène-glycol, le sorbitol et le polyéthylène-glycol.

31. Electrode adhésive destinée à établir un contact électrique avec une surface, comprenant:

un moyen de support et une borne pour établir une connexion avec un circuit électrique extérieur, et

un corps conducteur adhésif tel que défini dans la revendication 26 pour venir au contact de la surface avec laquelle doit être établi le contact électrique, le corps de trouvant au contact du moyen de support et étant en communication électrique avec la borne.

32. Electrode selon la revendication 31 dans laquelle la composition comprend, à titre des deux composants: l'acide 2-acrylamido-2-méthylpropane-sulfonique polymérisé réticulé, ses sels ou des copolymères de l'acide ou de ses sels comme premier composant, et un polymère hydrophile organique non réticulé comme second composant polymère, les proportions globales des premier et second composants étant d'environ 24% à 74% et de 1% à 40%, respectivement.

33. Electrode selon la revendication 32 dans laquelle le premier composant est une forme sel polymérisée de l'acide 2-acrylamido-2-méthylpropane-sulfonique.

34. Electrode selon la revendication 33 dans laquelle le sel est un sel de sodium.

35. Electrode selon l'une quelconque des revendications 32 à 34 dans laquelle le polymère hydrophile est un acide polyacrylique.

36. Electrode selon la revendication 35 dans laquelle le poids moléculaire de l'acide polyacrylique est d'environ 5000.

37. Electrode selon l'une quelconque des revendications 31 à 36 dans laquelle l'humectant est le glycérol.

38. Electrode selon l'une quelconque des revendications 31 à 36 dans laquelle l'humectant est le propylène-glycol, le sorbitol ou le polyéthylène-glycol.

39. Electrode selon l'une quelconque des revendications 31 à 38 dans lequel la quantité de l'humectant est d'environ 30%.

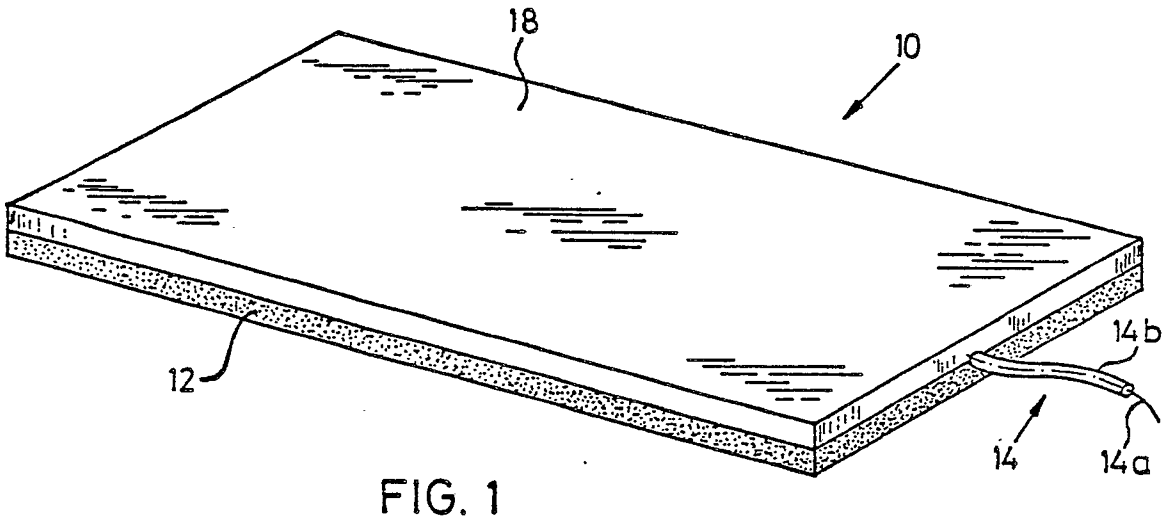


FIG. 1

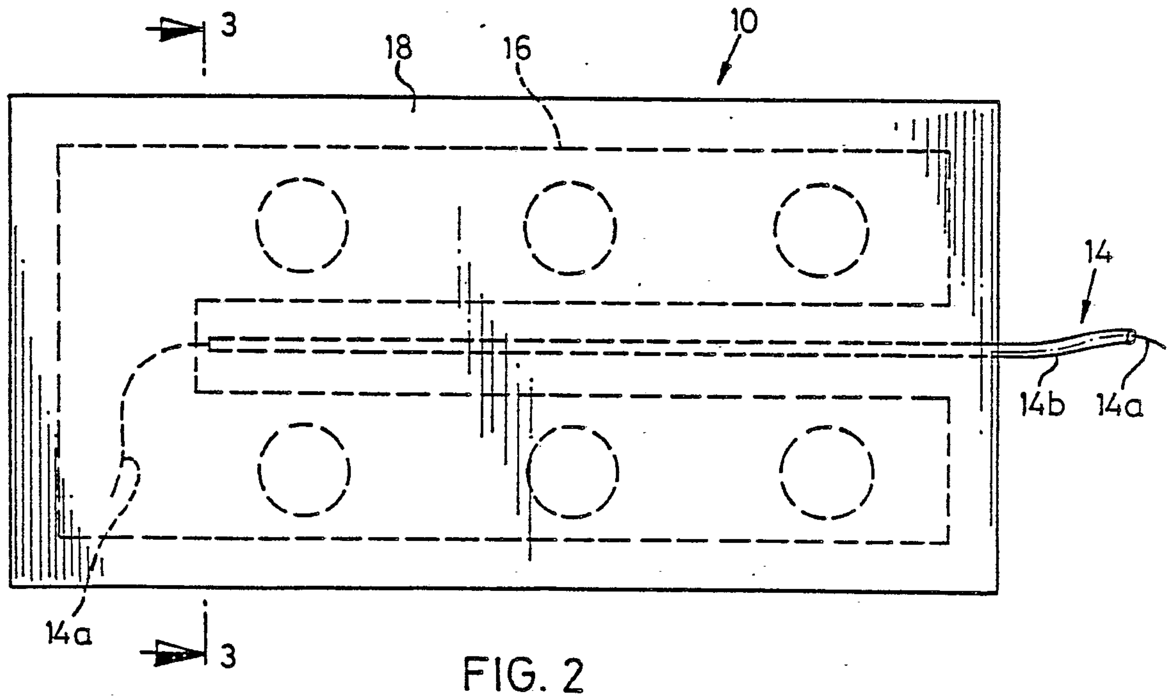


FIG. 2

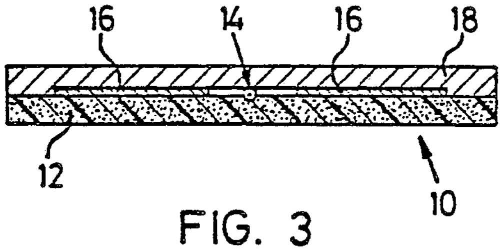


FIG. 3

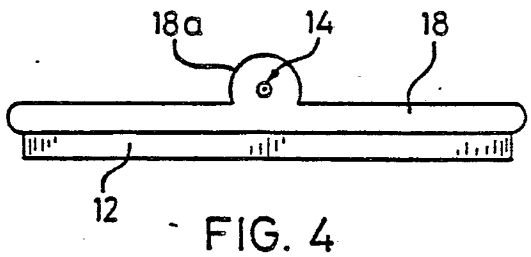


FIG. 4

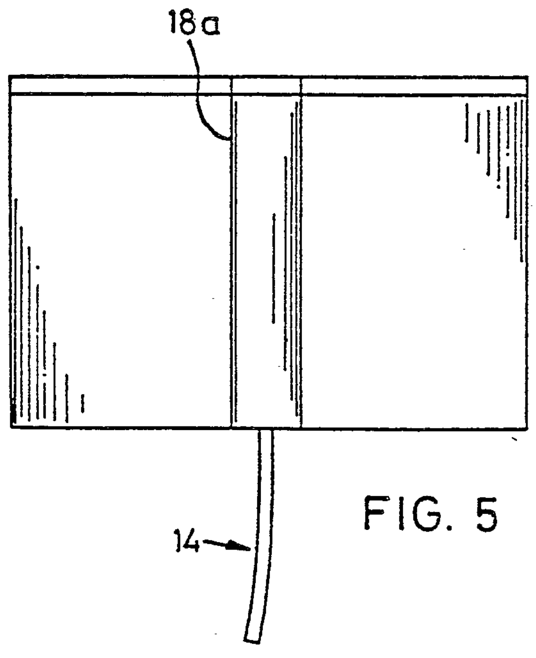


FIG. 5